(19) **Europäisches Patentamt / European Patent Office / Office européen des brevets**

(11) **EP 1 752 146 A1**

(12) **EUROPEAN PATENT APPLICATION**
published in accordance with Art. 158(3) EPC

(43) Date of publication:
**14.02.2007 Bulletin 2007/07**

(51) Int Cl.:
*A61K 31/198* (2006.01)   *A23L 1/305* (2006.01)
*A61K 31/401* (2006.01)   *A61K 31/4172* (2006.01)
*A61K 45/00* (2006.01)   *A61P 3/10* (2006.01)
*A61P 5/48* (2006.01)   *A61P 43/00* (2006.01)

(21) Application number: **05741366.8**

(22) Date of filing: **19.05.2005**

(86) International application number:
**PCT/JP2005/009126**

(87) International publication number:
**WO 2005/110394 (24.11.2005 Gazette 2005/47)**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **19.05.2004 JP 2004149320**

(71) Applicant: **Ajinomoto Co., Inc.**
**Tokyo 104-8315 (JP)**

(72) Inventors:
- **TSUJI, Mihoko,**
  **c/o AJINOMOTO CO., INC.**
  **Kawasaki-shi, Kanagawa 2108681 (JP)**
- **MITSUI, Akira,**
  **c/o AJINOMOTO CO., INC.**
  **Kawasaki-shi, Kanagawa 2108681 (JP)**

(74) Representative: **Nicholls, Kathryn Margaret et al**
**Mewburn Ellis LLP**
**York House**
**23 Kingsway**
**London WC2B 6HP (GB)**

(54) **THERAPEUTIC AGENT FOR DIABETES**

(57) The present invention provides an agent for recovering insulin secretion function which contains at least one of the amino acids selected from the group consisting of aspartic acid, cystine, serine, asparagine, isoleucine, histidine, hydroxyproline, glutamic acid, valine and salts thereof as an active ingredient; a functional food which contains the agent for recovering insulin secretion function; and a composition for treating diabetes which contains the amino acid(s) and a second diabetic drug as active ingredients. This agent for recovering insulin secretion function can be used as an oral therapeutic agent or functional food that is useful for treating, improving or preventing insulin secretion defects in Type II diabetes, borderline diabetes and other diseases, and side effects caused by administration of drugs.

EP 1 752 146 A1

**Description**

Technical Field of the Invention

The present invention relates to agents for recovering insulin secretion ability, and more specifically, it relates to the agents for recovering insulin secretion function that contain a specific amino acid(s) or salts thereof as an active ingredient. Further, the present invention relates to functional foods that contain the agents for recovering insulin secretion function; and compositions for treating diabetes that contain the specific amino acid(s) or the salts thereof and a diabetic drug(s).

Background of the Invention

Insulin-independent diabetes (Type II diabetes) is a disease wherein insulin is secreted but the blood glucose cannot be sufficiently controlled, and the drug therapy thereof is positioned as a treatment for patients whose conditions are not sufficiently improved by dietary therapy or exercise therapy. It is thought that causes thereof are decreased insulin secretion due to lowered insulin secretion function, lowered sugar uptake due to decreased sensitivity to insulin (increase of resistance), and the like. Up to now, agents have been developed such as preparations with insulin that is an endogenous hormone controlling hypoglycemic actions, or oral hypoglycemic agents having actions such as insulin secretagogue action or peripheral insulin sensitizing action. At present, it is the mainstream method of drug therapy of Type II diabetes that blood glucose is precisely controlled by using these oral hypoglycemic agents. However, all of the above therapeutic agents are insulin secretagogues of insulin itself or insulin secreting cells, and insulin sensitizers in insulin receptors. A drug has not yet been known that it can recover insulin secretion function of insulin secreting cells of which insulin secretion function has lowered.

Meanwhile, it is known that impaired glucose tolerance (IGT) occurs as a previous step of the development of Type II diabetes and it is also called as borderline diabetes. For instance, according to Japan Diabetes Society, borderline diabetes is defined as mild diabetes wherein the blood glucose level of 2 hours after the 75g sugar tolerance test is 140mg/dl or higher and less than 200mg/dl, and fasting blood glucose is 110mg/dl or higher and less than 126mg/dl (Non-patent Literature 1). Borderline diabetes is characterized by defective insulin secretion or insulin resistance. The same therapy with oral hypoglycemic agents or insulin preparations as mentioned above is conducted for preventing the transition from borderline diabetes to diabetes or treating IGT (borderline diabetes) (Non-patent Literature 2). However, a therapy with agents that can recover insulin secretion function has not yet been known.

On the other hand, it is known that insulin secretion function of pancreatic β cells is inhibited in case of polycystic ovarian syndrome (PCOS), acute pancreatitis, chronic pancreatitis or hemochromatosis, or in administering Ca antagonists, hypotensive diuretics such as thiazide agents, b-receptor blocking drugs such as carvedilol, steroids such as glucocorticoid, immunosuppressants such as tacrolimus, rapamycin and cyclosporine A, or $\alpha_2$ receptor stimulants such as clonidine. It is also known that the same oral hypoglycemic agents or insulin preparations as mentioned above are used for treating these diseases, but a therapy with agents that can recover insulin secretion function has not yet been known.

Meanwhile, several descriptions wherein amino acids are connected with diabetes have been known. For example, Patent Literature 1 discloses that L-leucine and L-arginine have endogenous insulin secretagogue action; Patent Literature 2 discloses that rise in the blood glucose levels is controlled when administering a composition comprised of an L-lysine hydrochloride, L-leucine, glycine, L-cystine hydrochloride and L-glutamic acid to rats that are diabetic models; and Non-patent Literature 3 discloses that an aspartic acid possibly has a function to increase insulin secretion. However, there is no description on specific amino acids that are effective against insulin secretion defects caused by diabetes or other diseases, or administration of specific drugs.

[Patent Literature 1] Japanese Patent Unexamined Publication No. Sho 60-255722
[Patent Literature 2] WO02/49636
[Non-patent Literature 1] Japan Diabetes Society, Tonyobyo Chiryo Gaido (Guide for treating diabetes) Bunkodo (1999)
[Non-patent Literature 2] Nippon Rinsho Shinjidai no tonyobyogaku 1 (Diabetology 1 of a new age) Nippon Rinsho (2002)
[Non-patent Literature 3] Metabolizm, Vol.49, 92-96, (2000)
[Non-patent Literature 4] H.A. A.M.A., Vol. 199, 519-524, (1967)
[Non-patent Literature 5] Diabetes Res Clin Pract., Vol.51, 1-8, (2001)

Disclosure of the Invention

The object of the present invention is to provide agents for recovering insulin secretion function.

The further object of the present invention is to provide oral therapeutic drugs or functional foods that are useful for treating, improving or preventing insulin secretion defects in Type II diabetes, borderline diabetes and other diseases, and side effects caused by administration of drugs.

The inventors thoroughly examined the above problems to solve them and found that aspartic acid, cystine, serine, asparagine, isoleucine, histidine, hydroxyproline, glutamic acid and valine, all of which are amino acids, have an excellent effect of recovering insulin secretion function. The present invention has been completed based on this finding.

Therefore, the present invention provides an agent for recovering insulin secretion function which contains at least one of the amino acids selected from the group consisting of aspartic acid, cystine, serine, asparagine, isoleucine, histidine, hydroxyproline, glutamic acid, valine and salts thereof as an active ingredient.

The present invention also provides an agent for treating diabetes which contains at least one of the amino acids selected from the group consisting of aspartic acid, cystine, serine, asparagine, isoleucine, histidine, hydroxyproline, glutamic acid, valine and salts thereof; and a second diabetic drug as active ingredients.

Further, the present invention provides a kit for treating diabetes which is comprised of a composition(s) having L- aspartic acid(s) or salts thereof as an active ingredient; and a diabetic drug(s) containing an insulin secretagogue(s).

Additionally, the present invention provides a functional food which contains an agent(s) for recovering insulin secretion function.

Brief Description of the Drawings

Fig. 1 shows a diagram indicating the recovering effect of aspartic acid on sugar tolerance.

Fig. 2 shows a diagram indicating the recovering effect of pancreatic β cells of aspartic acid on insulin secretion.

Best Mode for Carrying out the Invention

Agents for recovering insulin secretion function of the present invention can be used in improving insulin secretion defects caused by Type II diabetes, borderline diabetes and other diseases, and administration of drugs. More specifically, they can be used as agents for improving insulin secretion function, functional foods having the improving effect thereof, and the like. In the present invention, improvement of insulin secretion defects means recovery from the defects in insulin secretion function in insulin secretion cells such as pancreatic β cells. Treatment and prevention thereof are also included therein. The insulin secretion defects caused by other diseases include those in the polycystic ovarian syndrome (PCOS), acute pancreatitis, chronic pancreatitis or hemochromatosis. The insulin secretion defects caused by administration of drugs include those as side effects in administering the Ca antagonists, hypotensive diuretics such as thiazide agents, b-receptor blocking drugs such as carvedilol, steroids such as glucocorticoid, immunosuppressants such as tacrolimus, rapamycin and cyclosporine A, or $\alpha_2$ receptor stimulants such as clonidine.

In the present invention, when aspartic acid, cystine, serine, asparagine, isoleucine, histidine, hydroxyproline, glutamic acid, valine or salts thereof are used for improving insulin secretion defects in Type II diabetes, borderline diabetes and other diseases and administration of drugs, each may be used by itself or arbitrarily combined with each other. It is preferable to use aspartic acid, cystine, serine or asparagine, and particularly preferable to use an aspartic acid.

The aspartic acid, cystine, serine, asparagine, isoleucine, histidine, hydroxyproline, glutamic acid and valine used in the present invention are those known as amino acids. They may be in L-form, D-form, racemic form or combination of L-form and D-form, and L-form is preferable. These can be used without changing, and their hydrates, solvates or various salts can also be used. Precursors that resolve *in vivo* to form these amino acids may be used.

In the present invention, in case of using either one of or proper combination of aspartic acid, cystine, serine, asparagine, isoleucine, histidine, hydroxyproline, glutamic acid and valine to improve insulin secretion defects caused by Type II diabetes, borderline diabetes (impaired glucose tolerance), other diseases and administration of drugs, they can be administered orally, intravenously or transdermally. Though the dosage differs depending on a patient's symptom, age and administration method, it is usually 20mg/day to 50g/day, and preferably 0.1 to 20g/day. Among the amino acids used in the present invention, isoleucine and valine, which are essential amino acids, are preferably administered twice or more of those amino acids that human requires in a day, and particularly preferably in 10 to 200 times as much as the amount. Especially, in case of aspartic acid, the dosage is 15 to 40g/day.

The agents for recovering insulin secretion function of the present invention or functional foods that contain them can be formulated into a preparation by ordinary methods. The dosage forms are, for example, injection solvents, tablets, granules, subtle granules, powders, capsules, cream pharmaceuticals and suppositories. The preparation carriers include such as lactose, glucose, D-mannitol, starch, crystalline cellulose, calcium carbonate, kaolin, starch, gelatin, hydroxypropyl cellulose, hydroxypropyl methyl cellulose, polyvinylpyrrolidone, ethanol, carboxy methyl cellulose, carboxy methyl cellulose calcium salts, magnesium stearate, talc, acetyl cellulose, sucrose, titanium oxide, benzoic acid, p-hydroxybenzoate ester, sodium dehydroacetate, gum arabic, tragacanth, methyl cellulose, egg yolk, surfactants, sucrose, simple syrup, citric acid, distilled water, ethanol, glycerin, propylene glycols, macrogol, monobasic sodium phosphate, dibasic sodium phosphate, sodium phosphate, glucose, sodium chloride, phenol, thimerosal, p-hydroxybenzoate ester and acid sodium sulfite. They are used by being mixed with the above amino acids depending on the dosage forms.

Further, the content of the active ingredient (the above amino acids) in the preparation of the present invention significantly varies depending on the dosage forms and is not particularly limited. Generally, the content is about 0.01 to 100 wt%, and preferably 1 to 100 wt% to a total amount of compositions. Especially, it is preferable to contain 50 wt% or more of the active ingredient in case of the agents for recovering insulin secretion function.

On the other hand, when using aspartic acid, cystine, serine, asparagine, isoleucine, histidine, hydroxyproline, glutamic acid, valine or salts thereof in order to treat, improve or prevent the above diseases in the present invention, they can be used by being combined with a second diabetic drug, that is, another diabetic drug, especially an insulin secretory drug. Particularly, this is useful for patients with severe Type II diabetes since the combination thereof has a high hypoglycemic action without administering insulin.

In such a case, diabetic drugs (the second diabetic drug) combined with the aspartic acid, cystine, serine, asparagine, isoleucine, histidine, hydroxyproline, glutamic acid, valine or salts thereof include α-glucosidase inhibitors such as acarbose and voglibose; insulin sensitizers such as pioglitazone and rosiglitazone; sulfonylurea agents such as glibenclamide, gliclazide, glimepiride, tolbutamide, acetohexamide, tolazamide, glyclopyramide, and glybuzole; rapid-acting insulin secretagogues such as nateglinide, repaglinide and mitighnide; and biguanides such as metformin and buformin.

Their doses and usage can be arbitrarily determined corresponding to the symptoms so that they conform to the doses and usage of the amino acids of the present invention.

In the present invention, the above amino acids (and/or salts thereof) and a second diabetic drug can be combined in the arbitrary ratio. Here, the second diabetic drug can be used as one kind or the combination of two or more kinds of the above diabetic drugs.

For example, in case of L-aspartic acid and nateglinide, it is preferable to orally administer 20mg to 50g/day, preferably 0.1 to 40g/day of L-aspartic acid, and more preferably 3 to 10g of L-aspartic acid once to four times per day. Meanwhile, as for nateglinide, it is preferable to orally administer 30 to 360mg/day thereof, preferably 30 to 120mg thereof once to four times per day, and more preferably 60 to 120mg thereof three times per day before meals.

Namely, combination of the above amino acids (and/or salts thereof), especially L-aspartic acid and a second diabetic drug, especially insulin secretagogues such as nateglinide can accomplish a high hypoglycemic action to patients whose responsiveness of pancreatic β cells to particularly insulin secretagogues significantly lowers, such as patients with severe Type II diabetes, without administering insulin. Further, changes in the ratio of the above amino acids (and/or salts thereof) and a second diabetic drug can introduce most appropriate insulin secretion responsiveness corresponding to pathologies.

The agents for recovering insulin secretion function of the present invention improve insulin secretion defects caused by Type II diabetes, borderline diabetes, and other diseases and administration of drugs, and are useful for treating, improving and preventing these diseases. The agents for recovering insulin secretion function of the present invention can be used as agents for improving insulin secretion function.

Example 1

(Confirmation of the effect of improving insulin secretion defects)

INS-1 cells derived from pancreatic β cells of rats were dispersed in each amount of $2 \times 10^5$ cells on a 24-well culture plate, then divided into three groups and cultured under the following conditions. That is:

First group: Culture on RPMI medium (Non-patent Literature 4) under the ordinary condition. The concentrations of the amino acids in the RPMI medium are shown in Table 1.
Second group: Culture under the secretion defective condition by culture under the high glucose concentration. That is, the glucose concentration was increased under the culture conditions of the first group.
Third group: Under the culture condition of the second group, culture was conducted so that the end concentration of aspartic acid, cystine, serine, asparagine, isoleucine, histidine, hydroxyproline, glutamic acid or valine in the medium became 10 times as high as the concentration thereof in the RPMI medium.
First group: ordinary condition cultured in 11.1mM glucose for 28 hours, cultured in 11.1mM glucose for 19 hours
Second group: secretion defective condition cultured in 25mM glucose for 28 hours, cultured in 11.1mM glucose for 19 hours

Third group: secretion defective + amino acid condition cultured in 25mM glucose for 28 hours, cultured in 11.1mM glucose + amino acids for 19 hours

Table 1 Amino acid concentrations in the medium

| Amino acid | Concentration (mg/l) | Concentration (mM) |
|---|---|---|
| L-Arginine | 200 | 1.149 |
| L-Asparagine | 50 | 0.379 |
| L-Aspartic acid | 20 | 0.150 |
| L-Cystine dihydrochloride | 65 | 0.208 |
| L-Glutamic acid | 20 | 0.136 |
| L-Glutamine | 300 | 2.055 |
| Glycine | 10 | 0.133 |
| L-Histidine | 15 | 0.097 |
| L-Hydroxyproline | 20 | 0.153 |
| L-Isoleucine | 50 | 0.382 |
| L-Leucine | 50 | 0.382 |
| L-Lysine hydrochloride | 40 | 0.274 |
| L-Methionine | 15 | 0.101 |
| L-Phenylalanine | 15 | 0.091 |
| L-Proline | 20 | 0.174 |
| L-Serine | 30 | 0.286 |
| L-Threonine | 20 | 0.168 |
| L-Tryptophan | 5 | 0.025 |
| L-Tyrosine disodium,dihydrate | 29 | 0.111 |
| L-Valine | 20 | 0.171 |

Those cultured cells were further cultured for 1 hour in KRBH buffer (Non-patent Literature 5), and cultured for 2 hours in KRBH buffer containing 16.7mM glucose. Then, insulin secretion rate was determined. Under the secretion defective condition of the second group, the insulin secretion rate of the first group lowered to about 60% of that under the ordinary condition. The effect of improving insulin secretion defects of amino acids was calculated by the following index.

$$\text{Index of insulin secretion improvement} = ((\text{insulin secretion of the third group}) - (\text{insulin secretion of the second group}))/((\text{insulin secretion of the first group}) - (\text{insulin secretion of the second group}))$$

(Reference: The index of insulin secretion improvement becomes 0 when the insulin secretion in administration of amino acids is same as that in the secretion defective condition, and it becomes 1 when the insulin secretion in administration of amino acids is same as that in the ordinary condition.)

Results are shown in Table 2.

Table 2

| | Index of insulin secretion improvement |
|---|---|
| Ordinary cond. Secretion defective cond | 1.00 |
| | 0.00 |

(continued)

| | Index of insulin secretion improvement | |
|---|---|---|
| Secretion defective + amino acid cond. | Asp | 1.75 |
| Secretion defective + amino acid cond. | Cystine | 1.08 |
| Secretion defective + amino acid cond. | Ser | 0.97 |
| Secretion defective + amino acid cond. | Asn | 0.87 |
| Secretion defective + amino acid cond. | Ile | 0.84 |
| Secretion defective + amino acid cond. | His | 0.67 |
| Secretion defective + amino acid cond. | Hydroxyproline | 0.64 |
| Secretion defective + amino acid cond. | Glu | 0.57 |
| Secretion defective + amino acid cond. | Val | 0.35 |

As clarified in Table 2, insulin secretion was significantly improved ($p < 0.05$) in aspartic acid, cystine, serine, asparagine, isoleucine, histidine, hydroxyproline, glutamic acid and valine. Meanwhile, the same examination was conducted to lysine, leucine and arginine, but significant improvement in insulin secretion defects was not confirmed.

Example 2

(Improvement of glucose tolerance by aspartic acid)

Male GK rats (7 weeks old) were fed in the following groups.

CON group: 5 GK rats Controlled food was administered.
3AS group: 5 GK rats Food with 3% aspartic acid was administered.
6AS group: 5 GK rats Food with 6% aspartic acid was administered.

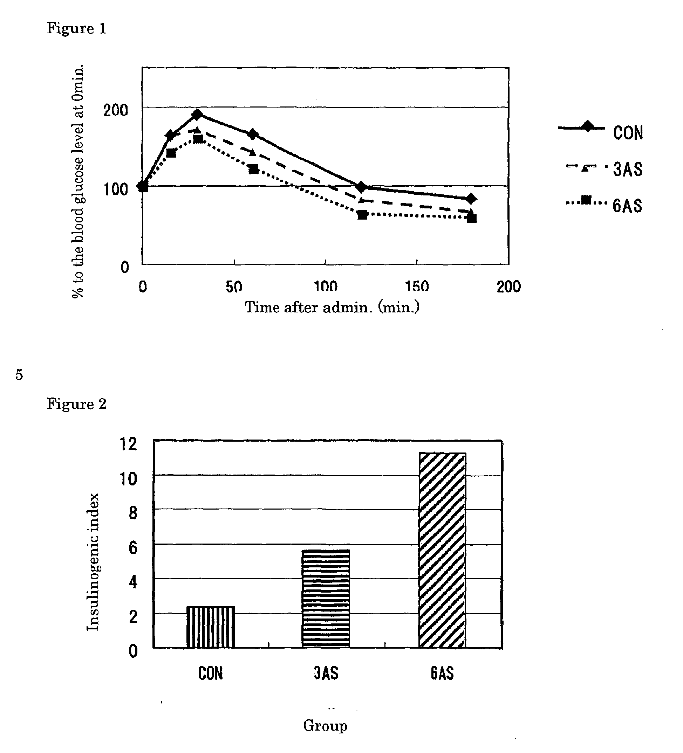

In the eighth week of administration, the glucose tolerance test (oral administration of 1g/kg of glucose) was conducted to the rats after 5 hour fasting, and their blood glucose and insulin levels were determined over time. Results were shown in Figure 1.
Each ∆AUC calculated from Figure 1 was 230 (mg/dl · h) in CON group, 190 (mg/dl · h) in 3AS group, and 140 (mg/dl - h) in 6AS group. After eight week administration, ∆AUC in the group to which 6% aspartic acid was administered clearly and significantly lowered and improvement in the glucose tolerance was seen.

Example 3

(The effect of improving insulin secretion of pancreatic β cells)

Insulinogenic index was determined from the results of the glucose tolerance test after eight week administration in Example 2. Insulinogenic index is the value wherein the insulin rate secreted in 15 minutes after adding glucose was divided by the glucose level that increased in 15 minutes after adding glucose. Results are shown in Figure 2.
As clarified in Figure 2, the insulinogenic index was improved corresponding to the administered dose in the groups to which the aspartic acid was administered. Thus, it is thinkable that administration of aspartic acid improved the insulin secretion function of pancreatic β cells.

Example 4

(The effect of combining aspartic acid with insulin secretagogues)

INS-1 cells derived from pancreatic β cells of rats were dispersed in each amount of $2 \times 10^5$ cells on a 24-well culture plate, and cultured on RPMI medium for 48 hours. After those cells were cultured for 1 hour in KRBH buffer, the culture was conducted for 2 hours under the following conditions, and insulin rate (insulin secretion rate) secreted in the medium was determined (n = 3).

Condition 1: KRBH buffer, 5mM glucose
Condition 2: KRBH buffer, 5mM glucose, 0.3 μ M nateglinide

Condition 3: KRBH buffer, 5mM glucose, 10mM aspartic acid (L-aspartic acid)
Condition 4: KRBH buffer, 5mM glucose, 0.3 $\mu$M nateglinide, 10mM aspartic acid

Each insulin secretion rate was $2.85\pm0.49$ ng/ml in Cond. 1, $4.34\pm1.19$ ng/ml in Cond. 2, $3.69\pm0.87$ ng/ml in Cond. 3, and $6.61\pm0.62$ ng/ml in Cond. 4. In case of nateglinide and aspartic acid by itself (Conditions 2 and 3), which are immediate-releasing insulin secretagogues, significant insulin secretion increase was not seen as compared to Condition 1. on the other hand, when combining nateglinide and aspartic acid (Condition 4), the significant insulin secretion increasing effect was seen as compared to either Conditions 1, 2, or 3 (p = 0.001, p = 0.043, p = 0.009).

These results indicate that, though insulin secretagogues can not increase insulin secretion of pancreatic $\beta$ cells, combination of insulin secretagogues and aspartic acid can increase insulin secretion.

Industrial Applicability

Aspartic acid, cystine, serine, asparagine, isoleucine, histidine, hydroxyproline, glutamic acid, valine or salts thereof by itself or those arbitrarily combined with each other can be used for improving insulin secretion ability of patients whose insulin secretion ability lowers because of Type II diabetes, borderline diabetes (impaired glucose tolerance) and other causes.

**Claims**

1. An agent for recovering insulin secretion function which contains at least one of the amino acids selected from the group consisting of aspartic acid, cystine, serine, asparagine, isoleucine, histidine, hydroxyproline, glutamic acid, valine and salts thereof as an active ingredient.

2. The agent for recovering insulin secretion function according to claim 1, wherein the aspartic acid, cystine, serine, asparagine, isoleucine, histidine, hydroxyproline, glutamic acid and valine are in L-form.

3. The agent for recovering insulin secretion function according to claim 2, wherein the amino acid is an L-aspartic acid or salt thereof.

4. A composition for treating diabetes which contains at least one of the amino acids selected from the group consisting of aspartic acid, cystine, serine, asparagine, isoleucine, histidine, hydroxyproline, glutamic acid, valine and salts thereof and a second diabetic drug as active ingredients.

5. The composition for treating diabetes according to claim 4, wherein the aspartic acid, cystine, serine, asparagine, isoleucine, histidine, hydroxyproline, glutamic acid and valine are in L-form.

6. The composition for treating diabetes according to claims 4 or 5, wherein the second diabetic drug is an insulin secretagogue.

7. The composition for treating diabetes according to claim 6 which contains L-aspartic acid or salt thereof, and an insulin secretagogue.

8. The composition for treating diabetes according to any one of claims 4 to 6, wherein two or more kinds of diabetic drugs are used as the second diabetic drug.

9. A kit for treating diabetes which is comprised of a composition having L-aspartic acid or salt thereof as an active ingredient; and a diabetic drug containing an insulin secretagogue.

10. A functional food which contains the agent for recovering insulin secretion function according to any one of claims 1 to 3.

11. Use of aspartic acid, cystine, serine, asparagine, isoleucine, histidine, hydroxyproline, glutamic acid, valine or salts thereof for preparing an agent for recovering insulin secretion function.

Figure 1

Figure 2

| INTERNATIONAL SEARCH REPORT | International application No. |
|---|---|
| | PCT/JP2005/009126 |

A.  CLASSIFICATION OF SUBJECT MATTER
    Int.Cl⁷  A61K31/198, A23L1/305, A61K31/401, 31/4172, 45/00, A61P3/10,
        5/48, 43/00

According to International Patent Classification (IPC) or to both national classification and IPC

B.  FIELDS SEARCHED

Minimum documentation searched (classification system followed by classification symbols)
    Int.Cl⁷  A61K31/198, A23L1/305, A61K31/401, 31/4172, 45/00, A61P3/10,
        5/48, 43/00

Documentation searched other than minimum documentation to the extent that such documents are included in the fields searched
    Jitsuyo Shinan Koho        1922-1996    Jitsuyo Shinan Toroku Koho    1996-2005
    Kokai Jitsuyo Shinan Koho  1971-2005    Toroku Jitsuyo Shinan Koho    1994-2005

Electronic data base consulted during the international search (name of data base and, where practicable, search terms used)
    CAplus(STN), MEDLINE(STN), BIOSIS(STN), EMBASE(STN), JSTPlus(JOIS)

C.  DOCUMENTS CONSIDERED TO BE RELEVANT

| Category* | Citation of document, with indication, where appropriate, of the relevant passages | Relevant to claim No. |
|---|---|---|
| E,X | JP 2005-132831 A  (Ajinomoto Co., Inc.), 26 May, 2005 (26.05.05), Full text (Family: none) | 10 |
| X | Shiro FUKUI et al., "Tonyobyo Kanja ni okeru Bunkisa Amino acids Fuka ga Insulin Bunpitsu ni Oyobosu Eikyo ni Kansuru Kenkyu", Rinsho to Kenkyu, 1989, Vol.66, No.3, pages 860 and 863 | 1,11 |
| X | FLATT, P.R. et al., Direct and indirect actions of nutrients in the regulation of insulin secretion from the pancreatic β cells, Proceedings of the Nutrition Society, 1991, Vol.50, pages 559 and 566 | 1,11 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 August, 2005 (16.08.05) | 06 September, 2005 (06.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/009126 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | KUHARA, T. et al., Effects of intravenous infusion of 17 amino acids on the secretion GH, glucagon, and insulin in sheep, Am.J. Physiol., 1991, Vol.260 (Endocrinol. Metab. 23), pages E21 and E26 | 1,2,11 |
| X | SJOHOLM, A., HISTAMINERGIC REGULATION OF PANCEREATIC β-CELL REPLICATION AND INSULIN SECRETION, BIOCHEMICAL AND BIOPHYSICAL RESEARCH COMMUNICATIONS, 1995, Vol.214, No.1, pages 224 and 229 | 1,2,11 |
| X | FLOYD, Jr., J.C. et al., Synergistic Effect of Essential Amino Acids and Glucose upon Insulin Sectretion in Man, DIABETES, 1970, Vol.19, pages 109 and 115 | 1,2,11 |
| X | FLATT, P.R. et al., Gastric Inhibitory Polypeptide and Insulin Responses to Orally Administered Amino Acids in Genetically Obese Hyperglycemic (ob/ob) Mice, J.Nutr., 1991, Vol.121, pages 1123 and 1128 | 1,2,11 |
| X | LEECH, C.A. et al., Regulation of Glucagon-Like Peptide-1 Receptor and Calcium-Sensing Receptor Signaling by L-Histidine, Endocrinology, 2003, Vol.144, No.11, pages 4851 and 4858 | 1,2,11 |
| A | YOSHIKAWA, Y. et al., New Insulinomimetic Zinc (II) Complexes of α-Amino Acids and Their Derivatives with Zn(N$_2$O$_2$) Coordination Mode, Chem.Pharm.Bull., 2001, Vol.49, No.5, pages 652 and 654 | 1-3,11 |
| A | SULOCHANA, K.N. et al., Beneficial role of amino acids in mitigating cytoskeletal actin glycation and improving F-actin content: In vitro, Glycoconjugate Journal, 2001, Vol.18, pages 277 and 282 | 1-3,11 |
| X | WO 2002/049636 A1 (THIRUVENGADAM, Rajagopal), 27 June, 2002 (27.06.02), Full text & AU 3597601 A | 4-6,8 |
| X | JP 60-255722 A (Otsuka Pharmaceutical Factory, Inc.), 17 December, 1985 (17.12.85), Full text (Family: none) | 4,5 |

Form PCT/ISA/210 (continuation of second sheet) (January 2004)

# EP 1 752 146 A1

| INTERNATIONAL SEARCH REPORT | International application No. |
|---|---|
| | PCT/JP2005/009126 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-500696 A  (Novo Nordisk A/S),<br>20 January, 1998 (20.01.98),<br>Claim 2<br>& WO 1995/032730 A1    & EP 760677 A1<br>& US 5652216 A | 4-9 |
| A | JP 7-118166 A  (Dott Research Laboratory),<br>09 May, 1995 (09.05.95),<br>Claims 2, 6<br>(Family: none) | 4-9 |
| A | JP 61-501569 A  (ERK, Vernon),<br>31 July, 1986 (31.07.86),<br>Full text<br>& WO 1985/003872 A1    & EP 174979 A1 | 4-9 |
| A | JP 61-501561 A  (ERK, Vernon),<br>31 July, 1986 (31.07.86),<br>Full text<br>& WO 1985/003870 A1    & EP 175714 A1 | 4-9 |
| A | JP 58-208218 A  (Snow Brand Milk Products<br>Co., Ltd.),<br>03 December, 1983 (03.12.83),<br>Full text<br>(Family: none) | 4-9 |
| X | JP 2003-171271 A  (Ajinomoto Co., Inc.),<br>17 June, 2003 (17.06.03),<br>Full text<br>(Family: none) | 10 |
| X | JP 2001-114681 A  (Ajinomoto Co., Inc.),<br>24 April, 2001 (24.04.01),<br>Full text<br>& US 6620967 B1 | 10 |
| X | JP 9-104624 A  (The Hokuren Federation of<br>Agricultural Cooperations, Japan),<br>22 April, 1997 (22.04.97),<br>Full text<br>(Family: none) | 10 |
| X | JP 7-252145 A  (Clintec Nutrition Co.),<br>03 October, 1995 (03.10.95),<br>Full text<br>& US 5756481 A         & US 5863906 A | 10 |
| X | JP 5-344863 A  (Meiji Milk Products Co., Ltd.),<br>27 December, 1993 (27.12.93),<br>Full text<br>(Family: none) | 10 |

Form PCT/ISA/210 (continuation of second sheet) (January 2004)

11

**INTERNATIONAL SEARCH REPORT**

| International application No. |
|---|
| PCT/JP2005/009126 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 60-1121 A  (Terumo Corp.),<br>07 January, 1985 (07.01.85),<br>Full text<br>(Family: none) | 10 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60255722 A

- WO 0249636 A

**Non-patent literature cited in the description**

- Japan Diabetes Society, Tonyobyo Chiryo Gaido. Bunkodo, 1999
- Nippon Rinsho Shinjidai no tonyobyogaku 1. Nippon Rinsho, 2002

- *Metabolizm,* 2000, vol. 49, 92-96
- *H.A. A.M.A.,* 1967, vol. 199, 519-524
- *Diabetes Res Clin Pract.,* 2001, vol. 51, 1-8